(19) Europäisches Patentamt
European Patent Office
Office européen des brevets

(11) Numéro de publication : **0 285 500 B1**

# (12) FASCICULE DE BREVET EUROPEEN

(45) Date de publication du fascicule du brevet : **02.01.92 Bulletin 92/01**

(51) Int. Cl.[5] : **C07D 233/32,** C07D 233/40, C07C 275/04, D06M 13/432

(21) Numéro de dépôt : **88400686.7**

(22) Date de dépôt : **22.03.88**

(54) **Urées substituées, leur procédé de préparation et leur application notamment dans l'ennoblissement des fibres cellulosiques.**

Le dossier contient des informations techniques présentées postérieurement au dépôt de la demande et ne figurant pas dans le présent fascicule.

(30) Priorité : **02.04.87 FR 8704629**

(43) Date de publication de la demande : **05.10.88 Bulletin 88/40**

(45) Mention de la délivrance du brevet : **02.01.92 Bulletin 92/01**

(84) Etats contractants désignés : **AT BE CH DE ES FR GB GR IT LI LU NL SE**

(56) Documents cités :
**EP-A- 0 141 755**
**DE-B- 1 172 265**
**FR-A- 1 558 244**
**KIRK-OTHMER: Encyclopedia of Chemical Technology, 3eme Ed., Vol. 22, John Wiley & Sons**
**AATCC Technical Manual**

(73) Titulaire : **SOCIETE FRANCAISE HOECHST**
**Société anonyme dite:**
**3, avenue du Général de Gaulle**
**F-92800 Puteaux (FR)**

(72) Inventeur : **Wilhelm, Didier**
**26 rue Diderot**
**F-92130 Issy Les Moulineaux (FR)**
Inventeur : **Gelabert, Antonio**
**9 allée Denis Papin**
**F-95570 Bouffemont (FR)**
Inventeur : **Blanc, Alain**
**21bis rue Galvanis**
**F-75017 Paris (FR)**

(74) Mandataire : **Rinuy, Santarelli**
**14, avenue de la Grande Armée**
**F-75017 Paris (FR)**

**EP 0 285 500 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des urées substituées, leur procédé de préparation et leur application notamment dans l'ennoblissement des fibres cellulosiques.

Les urées cycliques de structure imidazolidinique et particulièrement la dihydroxymethyl-1,3 dihydroxy-4,5 imidazolidinone-2 et la dihydroxy-4,5 diméthyl-1,3 imidazolidinone-2 sont des matières premières couramment employées pour la préparation d'apprêts textiles destinés à l'ennoblissement des fibres cellulosiques. Selon le brevet français n° 1.558.244, la dihydroxymethyl-1,3 dihydroxy-4,5 imidazolidinone-2 est obtenue en faisant réagir à un PH supérieur à 7, une mole d'urée avec au moins 0,8 mole de glyoxal et au moins 1,6 mole de formaldéhyde. Quant à la dihydroxy-4,5 diméthyl-1,3 imidazolidinone-2, elle peut être obtenue selon le brevet européen n° 0.141.755 en faisant réagir du glyoxal avec un excès ou non de diméthyl-1,3 urée, à un PH compris entre 4 et 7, en présence d'acide orthophosphorique.

La présente invention a pour objet les urées substituées de formule (I) sous leurs formes racémiques ou leurs mélanges de stéréoisomères

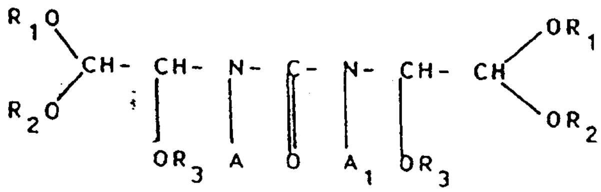

(I)

dans laquelle, soit $R_1$ et $R_2$, identiques, représentent un groupement $-CH_2$ R dans lequel R représente un atome d'hydrogène ou un radical alkyle en $C_1$-$C_4$, soit $R_1$ et $R_2$ forment ensemble un groupement $-CH_2$-$(CR_4R_4)_n$-$CH_2-$ dans lequel n représente 0 ou 1 et $R_4$ représente un atome d'hydrogène ou un groupement méthyle, $R_3$ représente un atome d'hydrogène ou un radical $CH_2R_5$ dans lequel $R_5$ représente un atome d'hydrogène ou un groupement alkyle en $C_1$-$C_4$ et soit A et $A_1$, identiques, représentent un atome d'hydrogène, soit A et $A_1$ forment ensemble un radical éthylène, triméthylène, $-CH(OCH_2R_6)$-$CH(OCH_2R_6)-$ dans lequel $R_5$ représente un atome d'hydrogène ou un groupement alkyle en $C_1$-$C_4$ ou, dans le cas où $R_3$ représente un atome d'hydrogène, un groupement dihydroxy-1,2 éthylène.

L'expression "alkyle en $C_1$-$C_4$" peut désigner, par exemple, un radical méthyle, éthyle, propyle, butyle, isopropyle, isobutyle.

L'expression "sous leurs formes racémiques ou leurs mélanges de stéréoisoméres" signifie que les atomes de carbone asymétriques des produits de formule (I) peuvent être sous leur configuration R et S.

L'invention a plus particulièrement pour objet les produits tels que définis ci-dessus, caractérisés en ce que, dans la formule (I), $R_1$ et $R_2$ sont identiques et représentent un groupement méthyle ou butyle, $R_3$ représente un atome d'hydrogène, un groupement méthyle ou butyle et, soit A et $A_1$, identiques, représentent un atome d'hydrogène, soit A et $A_1$ forment ensemble un groupement éthylène, diméthoxy-1,2 éthylène, dibutoxy-1,2 éthylène ou, dans le cas où $R_3$ représente un atome d'hydrogène, un groupement dihydroxy-1,2 éthylène.

Parmi ces produits, on peut citer plus particulièrement :

— la bis (diméthoxy-2,2 hydroxy-1 éthyl)-1,3 urée,
— la bis (triméthoxy-1,2,2 éthyl)-1,3 urée,
— la bis (dibutoxy-2,2 hydroxy-1 éthyl)-1,3 urée,
— la bis (tributoxy-1,2,2 éthyl)-1,3 urée,
— la bis (diméthoxy-2,2 hydroxy-1 éthyl)-1,3 imidazolidinone-2,
— la bis (triméthoxy-1,2,2 éthyl)-1,3 imidazolidinone-2,
— la bis (dibutoxy-2,2 hydroxy-1 éthyl)-1,3 imidazolidinone-2,
— la bis (tributoxy-1,2,2 éthyl)-1,3 imidazolidinone-2,
— la bis (diéthoxy-2,2 hydroxy-1 éthyl)-1,3 imidazolidinone-2,
— la bis (diméthoxy-2,2 hydroxy-1 éthyl)-1,3 dihydroxy-4,5 imidazolidinone-2,
— la bis (triméthoxy-1,2,2 éthyl)-1,3 diméthoxy-4,5 imidazolidinone-2,
— la bis (dibutoxy-2,2 hydroxy-1 éthyl)-1,3 dihydroxy-4,5 imidazolidinone-2,
— a bis (tributoxy-1,2,2 éthyl)-1,3 dibutoxy-4,5 imidazolidinone-2,
— la bis (diméthoxy-2,2 hydroxy-1 éthyl)-1,3 têtrahydro-1H pyrimidinone-2,
— la bis (triméthoxy-1,2,2 éthyl)-1,3 tétrahydro-1H pyrimidinone-2.

Selon l'invention, les produits de formule (I) ci-dessus peuvent être préparés par un procédé caractérisé

en ce que l'on fait réagir un éthanal disubstitué de formule (II) :

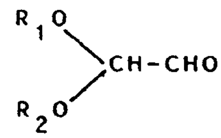 (II)

dans laquelle $R_1$ et $R_2$ ont la signification donnée précédemment avec une urée de formula (III) :

$$ANH\text{-}CO\text{-}NHA_1 \quad (III)$$

dans laqulle A et $A_1$ ont la signification donnée précédemment pour obtenir un produit de formule (IV) :

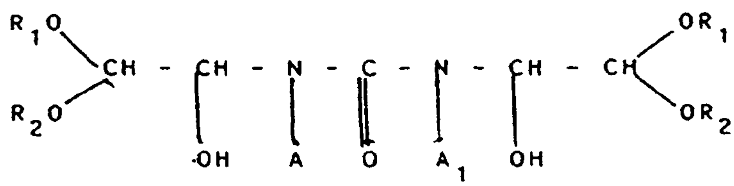 (IV)

(dans laquelle $R_1$, $R_2$, A et $A_1$ conservent la signification donnée plus haut) que l'on éthérifie ensuite, si on le desire, selon les methodes usuelles avec un alcool de formule (V) :

$$R_5CH_2OH \quad (V)$$

dans laquelle $R_5$ a la signification donnée précédemment.

Les urées de formule III sont des produits éoit commerciaux, soit dêcrits dans la littérature.

L'éthanal substitué de formule (II) peut être obtenu par le procédé décrit dans la demande de brevet français N° 86.07957, publiée sous le N° 2.599.362.

Dans des conditions préférentielles de mise en oeuvre du procédé de l'invention, la condensation du produit de formule (II) avec l'urée de formule (III) est réalisée :

— à pH alcalin, avantageusement à un pH compris entre 7 et 9, en présence d'un catalyseur alcalin compatible tel que l'hydroxyde de sodium ou l'hydroxyde de potassium ;

— à une température comprise entre 30 et 80°C.

Cette condensation peut être effectuée également dans l'eau ou dans un solvant organique compatible tel qu'un alcanol inférieur ou dans un milieu eau-alcanol inférieur. L'alcanol utilisé sera choisi de manière à éviter des réactions parasites de transéthérification.

Généralement, le procédé de l'invention est mis en oeuvre avec des quantités stoechiométriques de réactifs.

En fin de réaction de condensation, l'urée substituée de formule (IV) formée est soit isolée par des méthodes connues en elles-mêmes telles que la cristallisation, la chromatographie d'élution (cf W.C. STILL et al, J. Org. Chem., 1978, 43, 2923), soit elle est utilisée telle quelle, après neutralisation, si nécessaire, du catalyseur employé et élimination, si besoin est, du ou des solvants réactionnels par évaporation sous pression réduite. A l'état brut, l'urée substituée de formule (IV) présente une pureté suffisante pour être employée dans l'application décrite ci-après.

L'éthérification éventuelle des hydroxyles présents dans le produit de formule (IV) est effectuée selon les méthodes usuelles d'éthérification des groupements N-hydroxyméthylcarbamoyle par action d'un alcanol de formule (V) en milieu acide, avantageusement à un pH compris entre 2 et 6,5 et à une température comprise entre 20 et 80°C (cf. H. PETERSEN — Chemical Aftertreatment of Textiles, chapitre V, pages 135-265, H. Mark — Wiley Interscience 1971).

Les produits de formule (I) sous leurs formes racémiques ou leurs mélanges de stéréoisomères présentent d'intéressantes propriétés de réticulation de la cellulose et notamment, ils peuvent être utilisés pour conférer aux fibres cellulosiques des propriétés d'infroissabilité, d'irrétrécissabilité. Pour ces applications, ils peuvent être employés à des doses et sous des conditions opératoires similaires à celles couramment mises en oeuvre par l'homme du métier lorsqu'il utilise des résines textiles classiques à base, par exemple, de bis(hydroxyméthyl)-1,3 dihydroxy-4,5 imidazolidinone-2 (cf. H. PETERSEN, loc. cit.).

Mais dans ces applications d'ennoblissement des fibres cellulosiques, les produits de formule (I) présentent l'avantage de conférer aux fibres cellulosiques d'excellentes propriétés d'utilisation sans influencer trop défavorablement les propriétés mécaniques des fibres et, surtout, sans utiliser du formaldéhyde ou des composés susceptibles d'en libérer. En outre, les effets obtenus présentent une bonne permanence.

Les exemples suivants illustrent l'invention sans toutefois la limiter.

Les analyses physiques par spectroscopie de résonance magnétique nucléaire, RMN, soit du proton, $^1H$, soit du carbone treize, $^{13}C$, sont réalisées avec un appareil BRUCKER AC 200, à 200 Mz pour le proton et à 50 MHz pour le $^{13}C$. Les déplacements chimiques sont exprimés en ppm par rapport au tétraméthylsilane, TMS.

Certains stéréoisomères présentent de faibles différences de déplacements chimiques ; dans ce cas, ces déplacements chimiques ont été confirmés par l'enregistrement du spectre du mélange des stéréoisomères purifiés par voie physique.

Les chromatographies préparatives liquides à haute pression, HPLC, ont été réalisées à l'aide d'une pompe WATERS 600 et d'un réfractomètre différentiel WATERS 401 avec une colonne de 25 cm et de 2,54 mm de diamètre chargés avec soit du "LICHROSORB Si60" de Merck, soit du "POLYGOSIL C18" de Mascherey-Nagel.

## EXEMPLES

### Exemple 1 - Bis(diméthoxy-2,2 hydroxy-1éthyl)-1,3 urée.

On dissout sous agitation 24 g (0,4 mole) d'urée dans 96 g d'une solution méthanolique de diméthoxyéthanal à 87% en poids, soit 0,8 mole. La solution obtenue est ensuite amenée à pH = 8 par addition de quelques gouttes de soude 2N, puis elle est chauffée sous agitation deux heures à 50°C. On chasse ensuite le méthanol puis on reprend le résidu huileux par un mélange acétone-diéthyloxyde. Le produit cherché cristallise, on l'essore puis on le sèche sous vide à poids constant à 50°C.

On obtient ainsi la bis(diméthoxy-2,2 hydroxy-1 éthyl)-1,3 urée sous forme de prismes incolores, fondant à 90-110°C.

Microanalyse

| | | C % | H % | N % | O % |
|---|---|---|---|---|---|
| $C_9H_{20}N_2O_7$ | Calculés | 40,29 | 7,51 | 10,44 | 41,75 |
| P.M. = 268,27 | Trouvés | 40,8 | 7,8 | 10,6 | |

Analyses physiques

Ce produit soumis à une chromatographie préparative, HPLC, sur colonne de "POLYGOSIL C18" avec élution eau-méthanol, 96,5-3,5, conduit aux deux diastéréoisomères attendus : le premier isomère présente un temps de rétention, tr, de 244 s et le second de 635 s (débit 1 $cm^3$ par minute).

Les deux diastéréoisomères ainsi isolés présentent les spectres RMN$^1H$ suivants dans le diméthylsulfoxyde deutéré (DMSO $d_6$) :

| | 1er isomère | 2ème isomère |
|---|---|---|
| NH (d, 2H, J = 9,4 Hz) | 6,6 ppm | 6,6 ppm |
| OH (d, 2H, J = 5,6 Hz) | 5,53 ppm | 5,52 ppm |
| N-C<u>H</u>-O (m, 2H) | 5,02 ppm | 5,02 ppm |
| C<u>H</u> $(OMe)_2$ (d, 2H, J = 3,9 Hz) | 4,13 ppm | 4,14 ppm |
| OMe (s, 3H) | 3,34 ppm | 3,34 ppm |
| OMe (s, 3H) | 3,31 ppm | 3,31 ppm |

A la connaissance de la demanderesse, ces produits ne sont pas décrits dans la littérature.

### Exemple 2 - Bis(dibutoxy-2,2 hydroxy-1 éthyl)-1,3 imidazolidinone-2

On dissout sous agitation, à 40°C, 860 mg (10 mmoles) d'éthylènurée (imidazolidinone-2) et 3,76 g (20

mmoles) de dibutoxyéthanal dans 4 g d'acétone. La solution obtenue est ensuite amenée à pH légèrement alcalin par addition de quelques gouttes de soude 2N, puis elle est chauffée sous agitation 30 minutes à 30°C et enfin elle est concentrée sous vide. L'huile résiduelle est ensuite purifiée par chromatographie sur gel de silice avec élution à l'acétate d'éthyle. On isole ainsi deux fractions : la première contient un produit présentant un Rf de 0,6 et la seconde un produit de Rf 0,45.

Après élimination du solvant d'élution, on isole deux produits sous forme d'huile qui correspondent aux deux diastéréoisomères de la bis(dibutoxy-2,2 hydroxy-1 éthyl)-1,3 imidazolidinone-2 attendus.

Analyses physiques

$RMN^1H$, 200 MHz (DMSO $d_6$)

| | 1er isomère | 2ème isomère |
|---|---|---|
| OH (d, 2H, J = 6HZ) | 5,82 ppm | 5,79 ppm |
| N-CH-O (t, 2H, J = 6Hz) | 5,06 ppm | 5,06 ppm |
| $CH(OBu)_2$ (d, 2H, J = 6Hz) | 4,40 ppm | 4,36 ppm |
| $OCH_2$ et $NCH_2$ (m, 12H) | 3,6-3,23 ppm | 3,6-3,23 ppm |
| $CH_2$ (m, 16H) | 1,57-1,25 ppm | 1,53-1,24 ppm |
| $CH_3$ (m, 12H) | 0,92-0,83 ppm | 0,92-0,82 ppm |

$RMN^{13}C$, 200 MHz, (DMSO $d_6$)

| 1er isomère | | 2ème isomère | |
|---|---|---|---|
| 159,4 ppm | 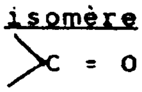 | 158,7 ppm | 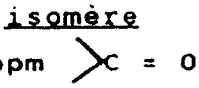 |
| 101,6 ppm | 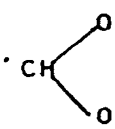 | 101,5 ppm | 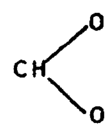 |
| 75,0 ppm | NHCHOH | 74,9 ppm | N-CHOH |
| 66,3 ppm | $O-CH_2-$ | 66,3 ppm | $O-CH_2-$ |
| 65,5 ppm | $O-CH_2-$ | 65,1 ppm | $O-CH_2-$ |
| 37,2 ppm | $N-CH_2-$ | 37,2 ppm | $N-CH_2-$ |
| 31,5 ppm | $-CH_2-$ | 31,5 ppm | $-CH_2-$ |
| 18,8 ppm | $-CH_2-$ | 18,8 ppm | $-CH_2-$ |
| 13,7 ppm | $-CH_3-$ | 13,7 ppm | $-CH_3-$ |

Spectres en accord avec la structure proposée. A la connaissance de la Demanderesse, ces produits ne sont pas décrits dans la littérature.

Exemple 3 - Bis(tributoxy-1,2,2 éthyl)-1,3 imidazolidinone-2

On dissout sous agitation à 40°C, 860 mg (10 mmoles) d'éthylèneurée et 3,765 g (20 mmoles) de dibutoxyéthanal dans 4 g d'acétone. La solution obtenue est ensuite amenée à pH légèrement alcalin par addition de quelques gouttes de soude 2N, puis elle est chauffée sous agitation 30 minutes à 30°C et enfin elle est concentrée sous vide. L'huile résiduelle est dissoute dans 16 g de n-butanol puis la solution obtenue est acidifiée avec quelques gouttes d'acide sulfurique à 50% dans l'eau et ensuite elle est abandonnée 2 heures sous agitation à la température ambiante avant d'être versée dans 100 g d'eau. On décante puis la phase aqueuse est lavée trois fois avec 50 g d'hexane, les phases organiques réunies sont ensuite lavées à la soude 1N puis à l'eau jusqu'à neutralité des lavages et, enfin, elles sont séchées sur chlorure de calcium anhydre, filtrées et

concentrées à sec sous vide. L'huile résiduelle brute est ensuite purifiée par chromatographie sur gel de silice avec élution à l'acétate d'éthyle. On obtient ainsi 3,43 g (6 mmoles) de bis(tributoxy-1,2,2 éthyl)-1,3 imidazolidinone-2, sous forme d'une huile incolore, soluble dans l'hexane et le diisopropyle oxyde.

Ce produit est un mélange des deux diastéréoisomères attendus.

Analyses physiques

1 - RMN$^1$H, $CDCl_3$ (chloroforme deutéré)

| | 1er isomère | 2ème isomère |
|---|---|---|
| N-CH-O (d, 2H) | 5,16 ppm (J=5,9Hz) | 5,15 ppm (J=5,6Hz) |
| $CH(OBu)_2$ (d, 2H) | 4,52 ppm (J=5,9Hz) | 4,51 ppm (J=5,6Hz) |
| $OCH_2$ et $NCH_2$ (m, 16H) | 3,63 - 3,41 ppm | |
| $CH_2$ (m, 24H) | 1,61 - 1,29 ppm | |
| $CH_3$ (m, 12H) | 0,95 - 0,86 ppm | |

2 - RMN$^{13}$C, $CDCl_3$

| | 1er isomère | 2ème isomère |
|---|---|---|
| >C = O | 160,42 ppm | 160,35 ppm |
| $-CH(OBu)_2$ | 100,8 ppm | 100,9 ppm |
| N - CH - OBu | 81,7 ppm | 81,8 ppm |
| $OCH_2$ | 68,1- 68,0- 67,1- 67,0 ppm<br>65,5 et 65,4 ppm | |
| N - $CH_2$ | 37,7 ppm | 37,9 ppm |
| $CH_2$ | 31,9- 31,8- 31,5- 19,3 ppm | |
| $CH_3$ | 13,8 ppm | |

A la connaissance de la Demanderesse, ces produits ne sont pas décrits dans la littérature.

Exemple 4 - Bis(diéthoxy-2,2 hydroxy-1 éthyl)-1,3 imidazolidinone-2

On chauffe une heure à 65 ± 5°C, sous agitation et à pH légèrement alcalin, 45 g (0,34 mole) de diéthoxyéthanal et 14,6 g (0,17 mole) d'éthylèneurée. Puis, après refroidissement, le produit cherché cristallise spontanément. On le recristallise dans le diisopropyle oxyde et on obtient ainsi 50,6 g (0,144 mole) de bis(diéthoxy-2,2 hydroxy-1 éthyl)-1,3 imidazolidinone-2, sous forme de cristaux incolores présentant un point de fusion de 105°C, soit un rendement de 85% de la théorie.

Microanalyse

| | | C % | H % | N % | O % |
|---|---|---|---|---|---|
| $C_{15}H_{30}N_2O_7$ | calculés | 51,41 | 8,63 | 8,0 | 31,96 |
| P.M. = 350,4 | trouvés | 51,3 | 8,7 | 8,0 | |

Ce produit analysé en chromatographie en couche mince sur gel de silice avec élution avec un mélange dichlorométhane-méthanol, 95-5, révèle la présence de deux produits présentant respectivement un Rf de 0,16 et de 0,21. Après isolement par HPLC préparative, ces deux produits présentent les spectres RMN suivants :

1 - RMN$^1$H, DMSO $d_6$

| | 1er isomère | 2ème isomère |
|---|---|---|
| OH (d, 2H, J = 6,0 HZ) | 5,85 ppm | 5,81 ppm |
| N-C<u>H</u>-O (t, 2H, J = 6,0 Hz) | 5,04 ppm | 5,04 ppm |
| C<u>H</u> $(OEt)_2$ (d, 2H, J = 6,0 Hz) | 4,41 ppm | 4,41 ppm |
| $-CH_2-$ (m, 12H) | 3,69 - 3,25 ppm | |
| $-CH_3-$ (m, 12H) | 1,17 - 1,05 ppm | |

2 - RMN$^{13}$C, DMSO $d_6$

| | 1er isomère | 2ème isomère |
|---|---|---|
| C = O | 159,4 ppm | 158,6 ppm |
| 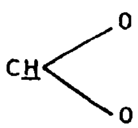 | 101,4 ppm | 101,2 ppm |
| N-CH-O | 75,1 ppm | 74,9 ppm |
| $OCH_2$ | 62,2 ppm | 62,2 ppm |
| $OCH_2$ | 61,4 ppm | 61,0 ppm |
| $N-CH_2$ | 37,1 ppm | 37,1 ppm |
| $CH_3$ | 15,3 ppm | 15,3 ppm |

A la connaissance de la Demanderesse, ce produit n'est pas décrit dans la littérature.

<u>Exemple 5 - Bis(diméthoxy-2,2 hydroxy-1 éthyl)-1,3 imidazolidinone-2</u>

On chauffe sous agitation une heure à 50°C une solution constituée de :

— 292,5 g (2 moles) de diméthoxyéthanal en solution à 53% en poids dans l'eau ;

— 86 g (1 mole) d'éthylèneurée ;

— une quantité suffisante de soude 2N pour obtenir un pH de 8,

puis la solution obtenue est concentrée sous vide. Le produit cherché cristallise spontanément. Après reprise à l'oxyde de diéthyle et séchage sous vide à poids constant à 40°C, on isole 262 g (0,89 mole) de bis (diméthoxy-2,2 hydroxy-1 éthyl)-1,3 imidazolidinone-2, cristallisée présentant un point de fusion de 127 ± 3°C.

Microanalyse

| | | C % | H % | N % | O % |
|---|---|---|---|---|---|
| $C_{11}H_{22}N_2O_7$ | calculés | 44,89 | 7,54 | 9,52 | 38,05 |
| P.M. = 294,31 | trouvés | 44,8 | 7,7 | 9,6 | |

RMN$^{13}$C, DMSO $d_6$

| | |
|---|---|
| C = O | 159,2 ppm |
| $—CH(OMe)_2$ | 103,1 ppm |
| N-CH-O | 74,5 ppm |
| $O-CH_3$ | 54,2 ppm |

| | |
|---|---|
| O-$CH_3$ | 53,6 ppm |
| N-$CH_2$ | 36,7 ppm |

A la connaissance de la Demanderesse, ce produit n'est pas décrit dans la littérature.

Exemple 6 - Bis(triméthoxy-1,2,2 éthyl)- 1,3 imidazolidinone-2

On chauffe 90 minutes à 50°C sous agitation une solution de 40,8 g (0,125 mole) de bis(diméthoxy-2,2 hydroxy-1 éthyl)-1,3 imidazolidinone-2 dans 85 g de méthanol et une quantité suffisante d'acide sulfurique à 25% en poids dans l'eau pour obtenir un pH de 4.

La solution obtenue est ensuite refroidie à la température ambiante, neutralisée à pH = 7 avec du bicarbonate de sodium et concentrée à sec sous vide. Le produit cristallisé obtenu est recristallisé par chaud et froid dans de l'oxyde de dipropyle.

On obtient ainsi 36,3 g (0,1125 mole) de bis (triméthoxy-1,2,2 éthyl)-1,3 imidazolidinone-2 cristallisée présentant un point de fusion de 66 ± 3°C.

Microanalyse

| | | C % | H % | N % | O % |
|---|---|---|---|---|---|
| $C_{13}H_{26}N_2O_7$ | calculés | 48,4 | 8,13 | 8,69 | 34,74 |
| P.M. = 322,4 | trouvés | 48,3 | 8,2 | 8,7 | |

A la connaissance de la Demanderessse, ce produit n'est pas décrit dans la littérature.

Exemple 7 - Bis(diméthoxy-2,2 hydroxy-1 éthyl)-1,3 dihydroxy-4,5 imidazolidinone-2

On dissout sous agitation à 50°C, 29,5 g (0,25 mole) de dihydroxy-4,5 imidazolidinone-2 isomère trans dans 104 g de diméthoxyéthanal en solution aqueuse à 50% en poids, soit 0,5 mole, puis la solution obtenue est amenée à pH = 8-9 par addition de quelques gouttes de soude 2N. Cette solution est ensuite chauffée une heure à 50°C sous agitation puis elle est concentrée sous vide.

L'huile résiduelle obtenue est purifiée ensuite par chromatographie sur gel de silice avec élution par un mélange dichlorométhane-méthanol. On obtient ainsi 40,7 g (0,125 mole) de bis(diméthoxy-2,2 hydroxy-1 éthyl)-1,3 dihydroxy-4,5 imidazolidinone-2 sous forme d'huile incolore dont les spectres RMN$^1$H et $^{13}$C sont en accord avec la structure proposée.

Microanalyse

| | | C % | H % | N % | O % |
|---|---|---|---|---|---|
| $C_{11}H_{22}N_2O_9$ | calculés | 40,49 | 6,79 | 8,59 | 44,13 |
| P.M. = 326,3 | trouvés | 39,8 | 6,9 | 8,4. | |

A la connaissance de la Demanderesse, ce produit n'est pas décrit dans la littérature.

Exemple 8

On imprègne au foulard, un tissu de popeline 100% coton, débouilli et blanchi, d'un poids d'environ 130 g par mètre carré, avec un taux d'exprimage de 75%, dans un bain aqueux contenant en solution :

— 0,4354 mole par litre d'une urée substituée selon la présente invention ;
— 12 g par litre de chlorure de magnésium hexahydraté ;
— 2,1 g par litre d'acide acétique ;
— 2 g par litre de nonyphénol éthoxylé avec 10 moles d'oxyde d'éthylène.

Le tissu est ensuite séché à 120°C puis il est soumis à un traitement thermique à 180°C durant 35 secondes sur une rame de laboratoire.

On détermine ensuite sur des échantillons du tissu traité :

— la défroissabilité selon la méthode AATCC N° 66-1978 sur des échantillons tels quels et sur des échantillons soumis à trois lavages ménagers à 60°C, la défroissabilité est exprimée par la somme des angles

de défroissement obtenus en sens chaîne et en sens trame ;

— la résistance à la traction, Rt, exprimée en daN, en sens chaîne plus en sens trame, selon la norme AFNOR G07001 ;

— les taux de formaldéhyde résiduel, Tfr, sur tissu par la méthode AATCC 112-1982 et parla méthode décrite dans la loi japonaise 112-1973.

Les résultats obtenus sont donnes dans le tableau I :

Tableau I

| | Angles de défroissement sur tissu tel quel | Angles de défroissement sur tissu après 3 lavages | Rt | Tfr |
|---|---|---|---|---|
| sur tissu non traité | 174 | 174 | 98,4 | non détectable |
| sur tissu traité avec le produit de l'exemple 5 | 273 | 248,5 | 63,1 | non détectable |
| sur tissu traité avec le produit de l'exemple 6 | 271 | 248 | 50,5 | non détectable |

On constate que les produits de la présente invention améliorent considérablement les qualités d'infroissabilité des tissus traités.

## Revendications

## Revendications pour les Etats contractants suivants : AT, BE, CH, DE, FR, GB, GR, IT, LI, LU, NL, SE

1. Urées substituées de formule (I) sous leurs formes racémiques ou leurs mélanges de stéréoisomères

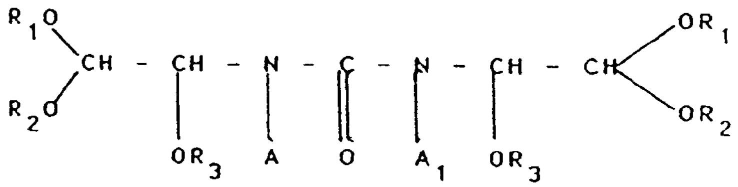

( I )

dans laquelle, soit $R_1$ et $R_2$, identiques, représentent un groupement —$CH_2$ R dans lequel R représente un atome d'hydrogène ou un radical alkyle en $C_1$-$C_4$, soit $R_1$ et $R_2$ forment ensemble un groupement —$CH_2$-$(CR_4R_4)_n$-$CH_2$ dans lequel n représente 0 ou 1 et $R_4$ représente un atome d'hydrogène ou un groupement méthyle, $R_3$ représente un atome d'hydrogène ou un radical —$CH_2$ $R_5$ dans lequel $R_5$ représente un atome d'hydrogène ou un groupement alkyle en $C_1$-$C_4$ et soit A et $A_1$, identiques, représentent un atome d'hydrogène, soit A et $A_1$ forment ensemble un radical éthylène, triméthylène, —$CH(OCH_2R_6)$-$CH(OCH_2R_6)$— dans lequel $R_5$ représente un atome d'hydrogène ou un groupement alkyle en $C_1$-$C_4$ ou, dans le cas où $R_3$ représente un atome d'hydrogène, un groupement dihydroxy-1,2 éthylène.

2. Urées telles que définies à la revendication 1, caractérisées en ce que dans la formule (I), $R_1$ et $R_2$ sont identiques et représentent un groupement méthyle ou butyle, $R_3$ représente un atome d'hydrogène, un groupement methyle ou butyle et soit A et $A_1$, identiques représentent un atome d'hydrogène, soit A et $A_1$ forment ensemble un groupement éthylène, diméthoxy-1,2 éthylène, dibutoxy-1,2 éthylène ou, dans le cas où $R_3$ représente un atome d'hydrogène, un groupement dihydroxy-1,2 éthylène.

3. Urée répondant à la formule (I) de la revendication 1, caractérisée en ce que c'est la bis (diméthoxy-2,2 hydroxy-1 éthyl)-1,3 urée.

4. Urée répondant à la formule (I) de la revendication 1, caractérisée en ce que c'est la bis (triméthoxy-1,2,2 éthyl)-1,3 urée.

5. Urée répondant à la formule (I) de la revendication 1, caractérisée en ce que c'est la bis (diméthoxy-2,2 hydroxy-1 éthyl)-1,3 imidazolidinone-2.

6. Urée répondant à la formule (I) de la revendication 1, caractérisée en ce que c'est la bis (triméthoxy-1,2,2 éthyl)-1,3 imidazolidinone-2.

7. Urée répondant à la formule (I) de la revendication 1, caractérisée en ce que c'est la bis (dibutoxy-2,2 hydroxy-1 éthyl)-1,3 imidazolidinone-2.

8. Urée répondant à la formule (I) de la revendication 1, caractérisée en ce que c'est la bis (tributoxy-1,2,2 éthyl)-1,3 imidazolidinone-2.

9. Urée répondant à la formule (I) de la revendication 1, caractérisée en ce que c'est la bis (diéthoxy-2,2 hydroxy-1 éthyl)-1,3 imidazolidinone-2.

10. Urée répondant à la formule (I) de la revendication 1, caractérisée en ce que c'est la bis (diméthoxy-2,2 hydroxy-1 éthyl)-1,3 dihydroxy-4,5 imidazolidinone-2.

11. Procédé de préparation des urées telles que définies par la formule (1) de la revendication 1, sous leurs formes racémiques ou leurs mélanges de stéréoisomères, caractérisé en ce que l'on fait réagir un éthanal disubstitué de formule (II) :

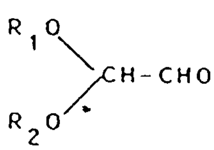

( I I )

dans laquelle $R_1$ et $R_2$ ont la signification selon la revendication 1 avec une urée de formule (III) :

$$ANH\text{-}CO\text{-}NHA_1 \quad (III)$$

dans laquelle A et $A_1$ ont la signification selon la revendication 1 pour obtenir un produit de formule (IV) :

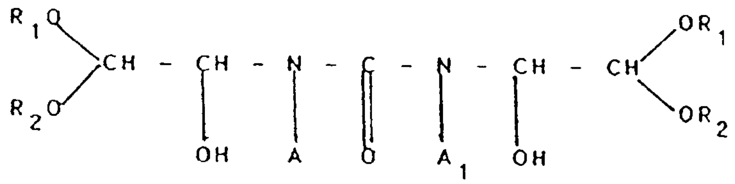

(IV)

dans laquelle $R_1$, $R_2$, A et $A_1$ conservent la signification donnée plus haut que, si désiré, on éthérifie selon les méthodes usuelles avec un alcool de formule (V) :

$R_5CH_2OH$ (V)

dans laquelle $R_5$ a la signification selon la revendication 1.

12. Application à titre de produits d'ennoblissement des fibres cellulosiques, des urées telles que définies par la formule (I) de la revendication 1, sous leurs formes racémiques et leurs mélanges de stéréoisomères.

13. Application à titre de produits d'ennoblissement des fibres cellulosiques, des urées telles que définies à la revendication 2, sous leurs formes racémiques et leurs mélanges de stéréoisomères.

14. Application à titre de produits d'ennoblissement des fibres cellulosiques, des urées telles que définies a l'une quelconque des revendications 3 à 10.

**Revendications pour l'Etat contractant suivant : ES**

1. Procédé de préparation des urées substituées de formule (I) sous leurs formes racémiques ou leurs mélanges de stéréoisomères

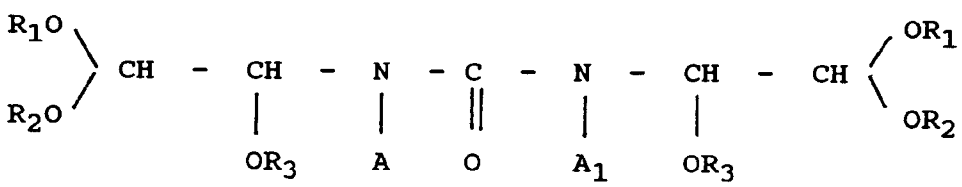

(I)

dans laquelle, soit $R_1$ et $R_2$, identiques, représentent un groupement —$CH_2R$ dans lequel R représente un atome d'hydrogène ou un radical alkyle en $C_1$-$C_4$, soit $R_1$ et $R_2$ forment ensemble un groupement —$CH_2$-$(CR_4R_4)_n$-$CH_2$ dans lequel n représente 0 ou 1 et $R_4$ représente un atome d'hydrogène ou un groupement méthyle, $R_3$ représente un atome d'hydrogène ou un radical —$CH_2R_5$ dans lequel $R_5$ représente un atome d'hydrogène ou un groupement alkyle en $C_1$-$C_4$ et soit A et $A_1$, identiques, représentent un atome d'hydrogène, soit A et Al forment ensemble un radical éthylène, triméthylène, —$CH(OCH_2R_5)$-$CH(OCH_2R_5)$— dans lequel $R_5$ représente un atome d'hydrogène ou un groupement alkyle en $C_1$-$C_4$ ou, dans le cas où $R_3$ représente un atome d'hydrogène, un groupement dihydroxy-1,2 éthylène, caractérisé en ce que l'on fait réagir un éthanal disubstitué de formule (II) :

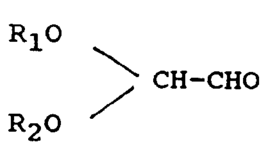

(II)

dans laquelle $R_1$ et $R_2$ ont la signification donnée plus haut avec une urée de formule (III) :

ANH-CO-$NHA_1$ (II)

dans laquelle A et $A_1$ ont la signification donnée plus haut pour obtenir un produit de formule (IV) :

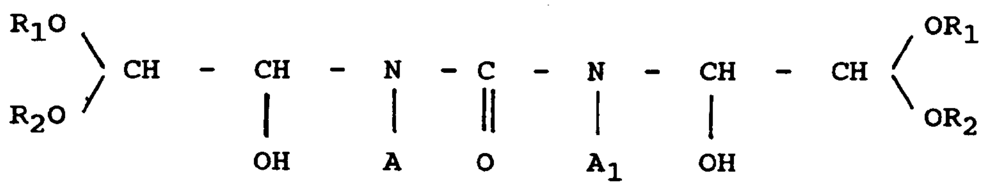 (IV)

dans laquelle $R_1$, $R_2$, A et $A_1$ conservent la signification donnée plus haut que, si désiré, on éthérifie selon les méthodes usuelles avec un alcool de formule (V) :

$$R_5CH_2OH \quad (V)$$

dans laquelle $R_5$ a la signification déjà indiquée.

2. Procédé selon la revendication 1, caractérisé en ce que dans la formule (I), $R_1$ et $R_2$ sont identiques et représentent un groupement méthyle ou butyle, $R_3$ représente un atome d'hydrogène, un groupement méthyle ou butyle et soit A et $A_1$ identiques représentent un atome d'hydrogène, soit A et $A_1$ forment ensemble un groupement éthylène, diméthoxy-1,2 éthylène, dibutoxy-1,2 éthylène ou, dans le cas où $R_3$ représente un atome d'hydrogène, un groupement dihydroxy-1,2 éthylène.

3. Procédé selon la revendication 1, caractérisé en ce que l'on prépare la bis (diméthoxy-2,2 hydroxy-1 éthyl)-1,3 urée.

4. Procédé selon la revendication 1, caractérisé en ce que l'on prépare la bis (triméthoxy-1,2,2 éthyl)-1,3 urée.

5. Procédé selon la revendication 1, caractérisé en ce que l'on prépare la bis (diméthoxy-2,2 hydroxy-1 éthyl)-1,3 imidazolidinone-2.

6. Procédé selon la revendication 1, caractérisé en ce que l'on prépare la bis (triméthoxy-1,2,2 éthyl)-1,3 imidazolidinone-2.

7. Procédé selon la revendication 1, caractérisé en ce que la bis (dibutoxy-2,2 hydroxy-1 éthyl)-1,3 imidazolidinone-2.

8. Procédé selon la revendication 1, caractérisé en ce que la bis (tributoxy-1,2,2 éthyl)-1,3 imidazolidinone-2.

9. Procédé selon la revendication 1, caractérisé en ce que la bis (diéthoxy-2,2 hydroxy-1 éthyl)-1,3 imidazolidinone-2.

10. Procédé selon la revendication 1, caractérisé en ce que la bis (diméthoxy-2,2 hydroxy-1 éthyl)-1,3 dihydroxy-4,5 imidazolidinone-2.

11. Application à titre de produits d'ennoblissemement des fibres cellulosiques, des urées telles que définies par la formule (I) de la revendication 1, sous leurs formes racémiques et leurs mélanges de stéréoisomères.

12. Application à titre de produits d'ennoblissement des fibres cellulosiques, des urées telles que définies à la revendication 2, sous leurs formes racémiques et leurs mélanges de stéréoisomères.

13. Application à titre de produits d'ennoblissement des fibres cellulosiques, des urées préparées selon l'une quelconque des revendications 3 à 10.

## Patentansprüche

## Patentansprüche für folgende Vertragsstaaten : AT, BE, CH, DE, FR, GB, GR, IT, LI, LU, NL, SE

1. Substituierte Harnstoffe der Formel (I) in ihren racemischen Formen oder als ihre Stereoisomerenmischungen

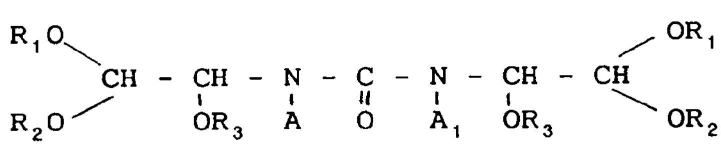 (I),

worin $R_1$ und $R_2$ gleich sind und eine Gruppe —$CH_2R$ darstellen, in der R ein Wasserstoffatom oder eine $C_1$-

$C_4$-Alkylgruppe ist, oder $R_1$ und $R_2$miteinander eine Gruppe —$CH_2(CR_4R_4)_n$-$CH_2$ bilden, in der n Null oder 1 ist und $R_4$ ein Wasserstoffatom oder eine Methylgruppe darstellt, $R_3$ ein Wasserstoffatom oder ein Rest —$CH_2R_5$ ist, in dem $R_5$ ein Wasserstoffatom oder eine $C_1$-$C_4$-Alkylgruppe bedeutet, und A und $A_1$ gleich sind und ein Wasserstoffatom darstellen oder A und $A_1$ miteinander Äthylen, Trimethylen oder —$CH(OCH_2R_6)$-$CH(OCH_2R_6)$— bilden, worin $R_6$ ein Wasserstoffatom, eine $C_1$-$C_4$-Alkylgruppe oder, wenn $R_3$ ein Wasserstoffatom ist, eine 1,2-Dihydroxyäthylengruppe bedeutet.

2. Harnstoffe nach Anspruch 1, dadurch gekennzeichnet, daß in Formel (I) $R_1$ und $R_2$ gleich sind und eine Methyl- oder Butylgruppe darstellen, $R_3$ ein Wasserstoffatom oder eine Methyl- oder Butylgruppe bedeutet und A und $A_1$ gleich sind und ein Wasserstoffatom darstellen oder A und $A_1$ miteinander eine Äthylen-, 1,2-Dimethoxyäthylen-, 1,2-Dibutoxyäthylen- oder, wenn $R_3$ ein Wasserstoffatom ist, eine 1,2-Dihydroxyäthylengruppe bilden.

3. Harnstoff der Formel (I) nach Anspruch 1, dadurch gekennzeichnet, daß er Bis-1,3-(2,2-dimethoxy-1-hydroxyäthyl)-harnstoff ist.

4. Harnstoff der Formel (I) nach Anspruch 1, dadurch gekennzeichnet, daß er Bis-1,3-(1,2,2-trimethoxyäthyl)-harnstoff ist.

5. Harnstoff der Formel (I) nach Anspruch 1, dadurch gekennzeichnet, daß er Bis-1,3-(2,2-dimethoxy-1-hydroxyäthyl)-2-imidazolidinon ist.

6. Harnstoff der Formel (I) nach Anspruch 1, dadurch gekennzeichnet, daß er Bis-1,3-(1,2,2-trimethoxyäthyl)-2-imidazolidinon ist.

7. Harnstoff der Formel (I) nach Anspruch 1, dadurch gekennzeichnet, daß er Bis-1,3-(2,2-dibutoxy-1-hydroxyäthyl)-2-imidazolidinon ist.

8. Harnstoff der Formel (I) nach Anspruch 1, dadurch gekennzeichnet, daß er Bis-1,3-(1,2,2-tributoxyäthyl)-2-imidazolidinon ist.

9. Harnstoff der Formel (I) nach Anspruch 1, dadurch gekennzeichnet, daß er Bis-1,3-(2,2-diäthoxy-1-hydroxyäthyl)-2-imidazolidinon ist.

10. Harnstoff der Formel (I) nach Anspruch 1, dadurch gekennzeichnet, daß er Bis-1,3-(2,2-dimethoxy-1-hydroxyäthyl)-4,5-dihydroxy-2-imidazolidinon ist.

11. Verfahren zur Herstellung von Harnstoffen der Formel (I) nach Anspruch 1 in ihren racemischen Formen oder als ihre Stereoisomerenmischungen, dadurch gekennzeichnet, daß man ein disubstituiertes Äthanal der Formel (II)

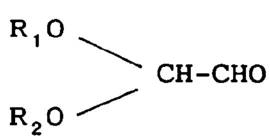

(II),

worin $R_1$ und $R_2$ die in Anspruch 1 angegebene Bedeutung haben, mit einem Harnstoff der Formel (III)

$$ANH\text{-}CO\text{-}NHA_1 \quad (III),$$

worin A und $A_1$ die in Anspruch 1 angegebene Bedeutung haben, zu einem Produkt der Formel (IV)

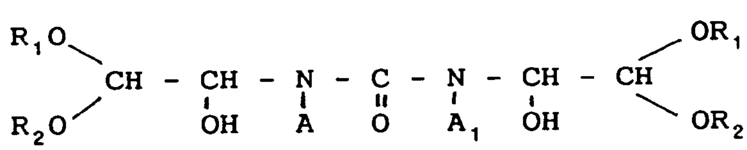

(IV),

worin $R_1$, $R_2$, A und $A_1$ die oben angegebene Bedeutung haben, umsetzt und dieses, wenn gewünscht, gemäß üblichen Verfahren mit einem Alkohol der Formel (V)

$$R_5CH_2OH \quad (V),$$

worin $R_5$ die in Anspruch 1 angegebene Bedeutung hat, veräthert.

12. Verwendung der Harnstoffe der Formel (I) nach Anspruch 1 in ihren racemischen Formen oder als ihre Stereoisomerenmischungen als Veredelungsmittel von Zellulosefasern.

13. Verwendung der Harnstoffe nach Anspruch 2 in ihren racemischen Formen oder als ihre Stereoisomerenmischungen als Veredelungsmittel von Zellulosefasern.

14. Verwendung der Harnstoffe nach einem der Ansprüche 3 bis 10 als Veredelungsmittel von Zellulosefasern.

**Patentansprüche für folgenden Vertragsstaat : ES**

1. Verfahren zur Herstellung von substituierten Harnstoffen der Formel (I) in ihren racemischen Formen oder als ihre Stereoisomerenmischungen

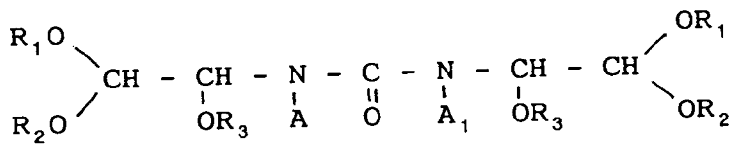 (I),

worin $R_1$ und $R_2$ gleich sind und eine Gruppe —$CH_2R$ darstellen, in der R ein Wasserstoffatom oder eine $C_1$-$C_4$-Alkylgruppe ist, oder $R_1$ und $R_2$ miteinander eine Gruppe —$CH_2$-$(CR_4R_4)_n$-$CH_2$ bilden, in der n Null oder 1 ist und $R_4$ ein Wasserstoffatom oder eine Methylgruppe darstellt, $R_3$ ein Wasserstoffatom oder ein Rest —$CH_2R_5$ ist, in dem $R_5$ ein Wasserstoffatom oder eine $C_1$-$C_4$-Alkylgruppe bedeutet, und A und $A_1$ gleich sind und ein Wasserstoffatom darstellen oder A und $A_1$ miteinander Äthylen, Trimethylen oder —$CH(OCH_2R_6)$-$CH(OCH_2R_6)$— bilden, worin $R_6$ ein Wasserstoffatom, eine$C_1$-$C_4$-Alkylgruppe oder, wenn $R_3$ ein Wasserstoffatom ist, eine 1,2-Dihydroxyäthylengruppe bedeutet, dadurch gekennzeichnet, daß man ein disubstituiertes Äthanal der Formel (II)

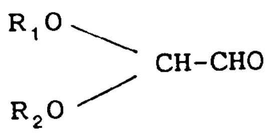 (II),

worin $R_1$ und $R_2$ oben angegebene Bedeutung haben, mit einem Harnstoff der Formel (III)

$$ANH\text{-}CO\text{-}NHA_1 \quad (III),$$

worin A und $A_1$ die oben angegebene Bedeutung haben, zu einem Produkt der Formel (IV)

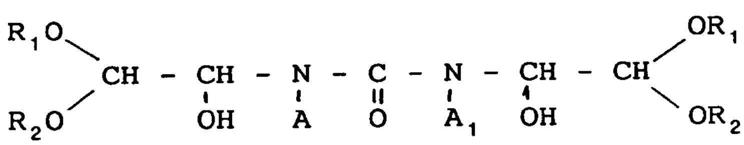 (IV),

worin $R_1$, $R_2$, A und $A_1$ die oben angegebene Bedeutung haben, umsetzt und dieses, wenn gewünscht, gemäß üblichen Verfahren mit einem Alkohol der Formel (V)

$$R_5CH_2OH \quad (V),$$

worin $R_5$ die bereits angegebene Bedeutung hat, veräthert.

2. Verfahren nach Anspruch 1, dadurch gekennzeichnet, daß in Formel (I) $R_1$ und $R_2$ gleich sind und eine Methyl- oder Butylgruppe darstellen, $R_3$ ein Wasserstoffatom oder eine Methyl- oder Butylgruppe bedeutet und A und $A_1$ gleich sind und ein Wasserstoffatom darstellen oder A und $A_1$ miteinander eine Äthylen-, 1,2-Dimethoxyäthylen-, 1,2-Dibutoxyäthylen- oder, wenn $R_3$ ein Wasserstoffatom ist, eine 1,2-Dihydroxyäthylengruppe bilden.

3. Verfahren nach Anspruch 1, dadurch gekennzeichnet, daß Bis-1,3-(2,2-dimethoxy-1-hydroxyäthyl)-harnstoff hergestellt wird.

4. Verfahren nach Anspruch 1, dadurch gekennzeichnet, daß Bis-1,3-(1,2,2-trimethoxyäthyl)-harnstoff hergestellt wird.

5. Verfahren nach Anspruch 1, dadurch gekennzeichnet, daß Bis-1,3-(2,2-dimethoxy-1-hydroxyäthyl)-2-imidazolidinon hergestellt wird.

6. Verfahren nach Anspruch 1, dadurch gekennzeichnet, daß Bis-1,3-(1,2,2-trimethoxyäthyl)-2-imidazolidinon hergestellt wird.

7. Verfahren nach Anspruch 1, dadurch gekennzeichnet, daß Bis-1,3-(2,2-dibutoxy-1-hydroxyäthyl)-2-imidazolidinon hergestellt wird.

8. Verfahren nach Anspruch 1, dadurch gekennzeichnet, daß Bis-1,3-(1,2,2-tributoxyäthyl)-2-imidazolidinon hergestellt wird.

9. Verfahren nach Anspruch 1, dadurch gekennzeichnet, daß Bis-1,3-(2,2-diäthoxy-1-hydroxyäthyl)-2-imidazolidinon hergestellt wird.

10. Verfahren nach Anspruch 1, dadurch gekennzeichnet, daß Bis-1,3-(2,2-dimethoxy-1-hydroxyäthyl)-4,5-dihydroxy-2-imidazolidinon hergestellt wird.

11. Verwendung von Harnstoffen, wie durch die Formel (I) von Anspruch 1 definiert, in ihren racemischen Formen oder als ihre Stereoisomerenmischungen als Veredelungsmittel von Zellulosefasern.

12. Verwendung von Harnstoffen, wie in Anspruch 2 definiert, in ihren racemischen Formen oder als ihre Stereoisomerenmischungen als Veredelungsmittel von Zellulosefasern.

13. Verwendung von Harnstoffen, hergestellt nach einem der Ansprüche 3 bis 10 als Veredelungsmittel von Zellulosefasern.

## Claims

**Claims for the following Contracting States : AT, BE, CH, DE, FR, GB, GR, IT, LI, LU, NL, SE**

1. Substituted ureas of formula I in their racemic forms or their stereoisomeric mixtures

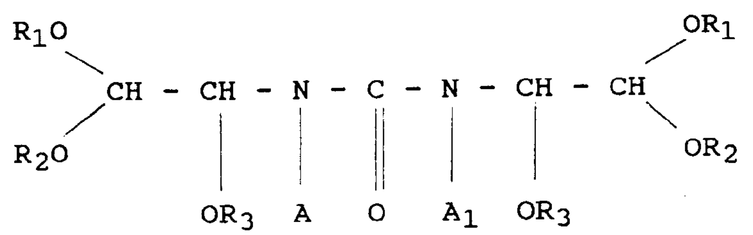

(I)

wherein either $R_1$ and $R_2$ are identical and denote a —$CH_2R$ group where R denotes a hydrogen atom or a $C_1$ to $C_4$ alkyl radical, or $R_1$ and $R_2$ together form a —$CH_2$-$(CR_4R_4)_n$-$CH_2$ group where n denotes 0 or 1 and $R_4$ denotes a hydrogen atom or a methyl group, $R_3$ denotes a hydrogen atom or a —$CH_2R_5$ radical where $R_5$ denotes a hydrogen atom or a $C_1$ to $C_4$ alkyl group and either A and $A_1$ are identical and denote a hydrogen atom, or A and $A_1$ together form an ethylene, trimethylene or —$CH(OCH_2R_5)$-$CH(OCH_2R_6)$ radical where $R_5$ denotes a hydrogen atom or a $C_1$ to $C_4$ alkyl group or, where $R_3$ denotes a hydrogen atom, a 1,2-dihydroxyethylene group.

2. Ureas as defined in claim 1, characterized in that in formula I $R_1$ and $R_2$ are identical and denote a methyl or butyl group, $R_3$ denotes a hydrogen atom, a methyl or butyl group, and either A and $A_1$ are identical and denote a hydrogen atom, or A and $A_1$ together form an ethylene group, a 1,2-dimethoxyethylene group, a 1,2-dibutoxyethylene group or, where $R_3$ denotes a hydrogen atom, a 1,2-dihydroxyethylene group.

3. A urea corresponding to formula I of claim 1, characterized in that it is bis-1,3-(2,2-dimethoxy-1-hydroxyethyl) urea.

4. A urea corresponding to formula I of claim 1, characterized in that it is bis-1,3-(1,2,2-trimethyoxyethyl) urea.

5. A urea corresponding to formula I of claim 1, characterized in that it is bis-1,3-(2,2-dimethoxy-1-hydroxyethyl)-2-imidazolidinone.

6. A urea corresponding to formula I of claim 1, characterized in that it is bis-1,3-(1,2,2-trimethoxyethyl)-2-imidazolidinone.

7. A urea corresponding to formula I of claim 1, characterized in that it is bis-1,3-(2,2-dibutoxy-1-hydroxyethyl)-2-imidazolidinone.

8. A urea corresponding to formula I of claim 1, characterized in that it is bis-1,3-(1,2,2-tributoxyethyl)-2-imidazolidinone.

9. A urea corresponding to formula I of claim 1, characterized in that it is bis-1,3-(2,2-diethoxy-1-hydroxyethyl)-2-imidazolidinone.

10. A urea corresponding to formula I of claim 1, characterized in that it is bis-1,3-(2,2-dimethoxy-1-hydroxyethyl)-4,5-dihydroxy-2-imidazolidinone.

11. A process for the preparation of ureas as defined by formula I in claim 1 in their racemic forms or their stereoisomeric mixtures, characterized in that a disubstituted ethanal of formula II :

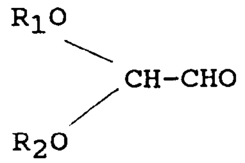

(II)

where $R_1$ and $R_2$ have the meaning stated in claim 1 is reacted with a urea of formula III :

$$ANH\text{-}CO\text{-}NHA_1 \quad (III)$$

where A and $A_1$ have the meaning stated in claim 1, to obtain a product of formula IV :

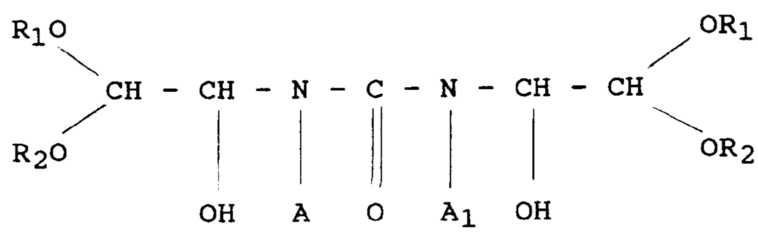

(IV)

where $R_1$, $R_2$, A and $A_1$ retain the meaning stated hereinbefore which, if required, is etherified by the usual methods with an alcohol of formula V :

$$E_5CH_2OH \quad (V)$$

where $R_5$ has the meaning stated in claim 1.

12. Application by way of products for improving cellulose fibres of the ureas as defined by formula I of claim 1, in their racemic forms and their stereoisomeric mixtures.

13. Application by way of products for improving cellulose fibres of the ureas as defined in claim 2, in their racemic forms and their stereoisomeric mixtures.

14. Application by way of products for improving cellulose fibres of the ureas as defined in any of claims 3 to 10.

**Claims for the following Contracting State : ES**

1. A process for the preparation of substituted ureas, as defined by formula (I), in their racemic forms or their stereoisomeric mixtures

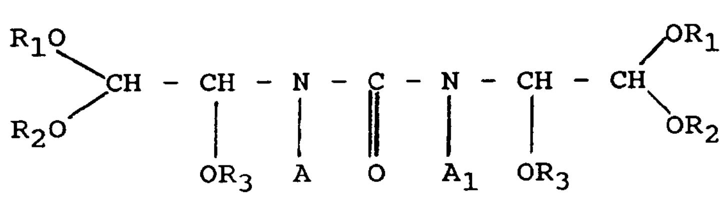

(I)

wherein either $R_1$ and $R_2$ are identical and denote a —$CH_2R$ group where R denotes a hydrogen atom or a $C_1$ to $C_4$ alkyl radical, or $R_1$ and $R_2$ together form a —$CH_2$-$(CR_4R_4)_n$-$CH_2$ group where n denotes 0 or 1 and $R_4$ denotes a hydrogen atom or a methyl group, $R_3$ denotes a hydrogen atom or a —$CH_2R_5$ radical where $R_5$ denotes a hydrogen atom or $C_1$ to $C_4$ alkyl group and either A and $A_1$ are identical and denote a hydrogen atom, or A and $A_1$ together form an ethylene, trimethylene or—$CH(OCH_2R_5)$-$CH(OCH_2R_5)$ radical where $R_5$ denotes

a hydrogen atom or a $C_1$ to $C_4$ alkyl group or, where $R_3$ denotes a hydrogen atom, a 1,2-dihydroxyethylene group, characterized in that a disubstituted ethanal of formula (II)

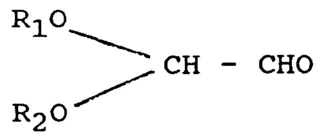 (II)

where $R_1$ and $R_2$ have the meaning stated hereabove is reacted with a urea of formula (III)

$$ANH\text{-}CO\text{-}NHA_1 \quad (III)$$

where A and $A_1$ have the meaning stated hereabove to obtain a product of formula (IV)

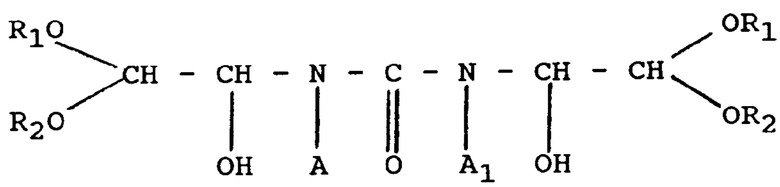 (IV)

where $R_1$, $R_2$, A and $A_1$ retain the meaning stated hereinbefore which, if required, is etherified by the usual methods with an alcohol of formula (V)

$$R_5CH_2OH \quad (V)$$

where $R_5$ has the meaning stated hereinbefore.

2. A process, as defined in claim 1, characterized in that in formula (I) $R_1$ and $R_2$ are identical and denote a methyl or butyl group, $R_3$ denotes a hydrogen atom, a methyl or butyl group, and either A and $A_1$ are identical and denote a hydrogen atom, or A and $A_1$ together form an ethylene group, a 1,2-dimethoxyethylene group, a 1,2-dibutoxyethylene group or, where $R_3$ denotes a hydrogen atom, a 1,2-dihydroxyethylene group.

3. A process, as defined in claim 1, characterized in that a bis-1,3-(2,2-dimethoxy-1-hydroxyethyl) urea is prepared.

4. A process, as defined in claim 1, characterized in that a bis-1,3-(1,2,2-trimethoxyethyl) urea is prepared.

5. A process, as defined in claim 1, characterized in that a bis-1,3-(2,2-dimethoxy-1-hydroxyethyl)-2-imidazolidinone is prepared.

6. A process, as defined in claim 1, characterized in that a bis-1,3- (1,2,2-trimethoxyethyl)-2-imidazol idinone is prepared.

7. A process, as defined in claim 1, characterized in that a bis-1,3-(2,2-dibutoxy-1-hydroxyethyl)-2-imidazolidinone is prepared.

8. A process, as defined in claim 1, characterized in that a bis-1,3-(1,2,2-tributoxyethyl)-2-imidazolidinone is prepared.

9. A process, as defined in claim 1, characterized in that a bis-1,3-(2,2-diethoxy-1-hydroxyethyl)-2-imidazol idinone is prepared ;

10. A process, as defined in claim 1, characterized in that a bis-1,3-(2,2-dimethoxy-1-hydroxyethyl)-4,5-dihydroxy-2-imidazolidinone is prepared.

11. Application by way of products for improving cellulose fibres of the ureas as defined by formula (I) of claim 1, in their racemic forms and their stereoisometric mixtures.

12. Application by way of products for improving cellulose fibres of the ureas as defined in claim 2, in their racemic forms and their stereoisometric mixtures.

13. Application by way of products for improving cellulose fibres of the ureas as defined in any of claims 3 to 10.